# EUROPEAN PATENT APPLICATION

(11) **EP 3 501 448 A1**
(43) Date of publication of application: **26.06.2019**
(21) Application number: 17841484.3
(22) Date of filing: 14.08.2017
(51) Int. Cl.: A61C 8/00

(54) **DENTAL IMPLANT DEVICE**

(30) Priority: 16.08.2016 JP 2016159745
(71) Applicant: Organ Technologies, Inc., Minato-ku Tokyo 105-0001 (JP); RIKEN, Wako-shi, Saitama 351-0198 (JP); National University Corporation Okayama University, Kita-ku Okayama-shi Okayama 700-8530 (JP)
(72) Inventor: TSUJI, Takashi, Wako-shi Saitama 351-0198 (JP); OSHIMA, Masamitsu, Okayama-shi Okayama 700-8530 (JP); KUBOKI, Takuo, Okayama-shi Okayama 700-8530 (JP); ANDO, Yoshiki, Kyoto-shi Kyoto 612-8501 (JP); OGAWA, Miho, Tokyo 105-0001 (JP)
(74) Representative: Weickmann & Weickmann PartmbB
(86) International application number: PCT/JP2017/029262
(87) International publication number: WO 2018/034262

(57) **Abstract**

The present invention provides a dental implant device comprising a dental implant body and an implant fixture. Said implant fixture is characterized in that it comprises a portion to be fixed to said implant body and a portion to be fixed to a tooth adjacent to the transplantation site of said implant body in a subject, and is configured so that mechanical stimulation from the tooth adjacent to said transplantation site is transmissible to said implant body, and said implant device is characterized in that it is placed into the oral cavity of said subject so that said implant body is inserted at said transplantation site while said implant fixture is fixed to said implant body and the tooth adjacent to said transplantation site.

## Description

### Technical Field

The present invention relates to a dental implant device that allows formation of functional periodontium.

### Background Art

Various therapeutic means are known for regaining the function of teeth lost by dental caries or periodontal disease. For example, a method of burying an artificial tooth produced by an artificial material such as metal or ceramics in the tooth root is known. Moreover, for example when the loss is completely to the tooth root, a method of bridging with a healthy tooth while placing an artificial tooth is known.

Further, in recent years, oral cavity implant therapy has been implemented as one of the cutting-edge therapeutic methods of this dental substitution medical care. The oral cavity implant therapy is a means of implanting an artificial tooth root such as titanium in the jaw bone of the site of tooth loss.

However, there is a major difference between the tooth root of a natural tooth and a dental implant (artificial tooth root). The difference is that the tooth root of a natural tooth is covered with periodontal membrane which is a part of the periodontium, whereas the transplantation site of the dental implant ordinarily does not have a periodontal membrane.

Fibrous periodontal membrane tissue which connects the cementum on the tooth root side and the alveolar bone on the outside exists around natural teeth. The cementum has the function to protect the tooth root surface and to attach the periodontal membrane to the tooth root surface. Moreover, the periodontal membrane is known to have three functions generally divided into 1) buffering action of occlusal force, 2) mobile capability of tooth (mechanics employed for orthodontic therapy etc.), and 3) neurotransmission function for transmitting noxious stimulation (such as pain stimulation) such as occlusion and orthodontal correction to the central nervous system. Among these, it is known that the periodontal membrane in particular has fibers that run vertical against the longitudinal direction of the tooth root in order to buffer the occlusal force of teeth, and this run of fibers in the periodontal membrane tissue is a configuration essential for functional expression of the periodontal membrane.

However, when an ordinary dental implant is transplanted, functional periodontium that exists around a natural tooth cannot be formed at the implant transplantation site. For this reason, there was a problem that absorption of the alveolar bone that supports the dental implants occurs at their transplantation sites due to occlusal force during long-term use, thus making them intolerable for use.

So far, it has been proposed to form functional periodontium on the implant surface after implant transplantation by placing a tooth germ tissue-derived or periodontal membrane tissue-derived cell mass on the dental implant surface for subjecting to transplantation (Patent Literature 1).

### Citation List

[Patent Literature 1] WO2013/115128

### Summary of the Invention

### Problems to be Solved by the Invention

Although the technology described in above Patent Literature 1 has potential as a technology that can form functional periodontium on the implant surface after implant transplantation, in order to utilize this technology, it was necessary to prepare a tooth germ tissue-derived or periodontal membrane tissue-derived cell mass in advance.

### Means for Solving the Problems

As a result of extensive investigation by the present inventors for a simpler method of forming functional periodontium on the implant surface after implant transplantation, the present invention has come to be completed.

In other words, in one embodiment, the present invention relates to a dental implant device that allows formation of functional periodontium, characterized in that:
said implant device comprises a dental implant body and an implant fixture,
said implant fixture comprises a portion to be fixed to said implant body and a portion to be fixed to a tooth adjacent to the transplantation site of said implant body in a subject, and is configured so that mechanical stimulation from the tooth adjacent to said transplantation site is transmissible to said implant body, and
said implant device is placed into the oral cavity of said subject so that said implant body is inserted at said transplantation site while said implant fixture is fixed to said implant body and the tooth adjacent to said transplantation site.

Moreover, in one embodiment, the device of the present invention is characterized in that said transplantation site is an extraction socket.

Moreover, in one embodiment, the device of the present invention is characterized in that said extraction socket is an extraction socket within seven days of tooth extraction.

Moreover, in one embodiment, the device of the present invention is characterized in that said implant body is inserted in said extraction socket so as not to contact the bottom of said extraction socket and is fixed by said implant fixture, to thereby maintain a state where said implant body does not contact the bottom of said extraction socket.

Moreover, in one embodiment, the device of the present invention is characterized in that said implant fixture is fixed to teeth on both sides adjacent to the transplantation site of said implant body.

Moreover, in one embodiment, the device of the present invention is characterized in that the material of said implant fixture is selected from the group consisting of a metal material, a ceramic material, a polymeric material, a hard fiber material, and ebonite.

Moreover, in one embodiment, the device of the present invention is characterized in that a periodontal membrane tissue-derived or tooth germ tissue-derived cell mass is placed on all or a part of the surface of said implant body.

Moreover, in one embodiment, the device of the present invention is characterized in that said tooth germ tissue-derived cell mass is a tooth germ mesenchymal tissue-derived or dental follicle tissue-derived cell mass.

Moreover, in one embodiment, the device of the present invention is characterized in that said cell mass is a periodontal membrane tissue-derived cell sheet.

Moreover, in one embodiment, the device of the present invention is characterized in that it further comprises a coating layer of a surface coating agent on the whole surface of said implant body or a portion thereof.

Moreover, in one embodiment, the device of the present invention is characterized in that said surface coating agent is selected from the group consisting of hydroxyapatite, α-tricalcium phosphate, β-tricalcium phosphate, and collagen.

Moreover, another embodiment of the present invention relates to a method of transplanting a dental implant to a subject comprising:
(A) a step of preparing a dental implant device that allows formation of functional periodontium,
   wherein said implant device comprises a dental implant body and an implant fixture, and
   said implant fixture comprises a portion to be fixed to said implant body and a portion to be fixed to a tooth adjacent to the transplantation site of said implant body in a subject, and is configured so that mechanical stimulation from the tooth adjacent to said transplantation site is transmissible to said implant body; and
(B) a step of placing said implant device into the oral cavity of said subject,
   wherein said implant device is placed into the oral cavity of said subject so that said implant body is inserted at said transplantation site while said implant fixture is fixed to said implant body and the tooth adjacent to said transplantation site.

Moreover, in one embodiment, the method of the present invention is characterized in that said transplantation site is an extraction socket within seven days of tooth extraction.

Moreover, in one embodiment, the method of the present invention is characterized in that in said step (B), said implant body is inserted in said extraction socket so as not to contact the bottom of said extraction socket and is fixed by said implant fixture, to thereby maintain a state where said implant body does not contact the bottom of said extraction socket.

Moreover, in one embodiment, the method of the present invention is characterized in that said subject is a non-human mammal.

An invention of any combination of one or more characteristics of the present invention described above is also encompassed by the scope of the present invention.

### Brief Description of the Drawings

Figure 1 shows the tissue images after tooth extraction of mandibular second molar (M2) in mouse jaw bone.
Figure 2 shows the design plan of the implant fixture employed in the Examples.
Figure 3 shows the dental implant device (dental implant and implant fixture) employed in the Examples.
Figure 4 shows an installation example of the dental implant device of the present invention to the extraction socket of mouse mandibular M2 tooth extraction model.
Figure 5 shows the micro CT images showing the change in tissue over time after transplanting an implant body with the dental implant device of the present invention. On transplantation Day 30 (about 4 w), distinct periodontal space is recognized between the implant superficial layer and the surrounding alveolar bone.
Figure 6 shows the tissue image (HE staining image) around the implant body at about 4 weeks after transplanting an implant body with the dental implant device of the present invention. Tissue structure equivalent to the periodontium of a natural tooth such as the cementum, the periodontal membrane, and the alveolar bone is recognized on the superficial layer of the implant body, showing that it is surviving on the jaw bone.
Figure 7 shows the tissue image (azan staining image) around the implant body at about 4 weeks after transplanting an implant body with the dental implant device of the present invention. The presence of distinctly running periodontal membrane fibers that connect the superficial layer of the implant body with the surrounding alveolar bone is shown.
Figure 8 shows the schematic diagram of a method of recovering biogenic periodontal membrane cells performed in Example 2.
Figure 9 shows the tissue images of cells obtained by dispersing the periodontal membrane cells recovered from a living body, seeding in a cell culture dish, and culturing.
Figure 10 shows the tissue image of the periodontal membrane cell sheet produced in Example 2.
Figure 11 shows the periodontal membrane cell sheet produced in Example 2 (left figure), and a photograph of wrapping said periodontal membrane cell sheet around a dental implant body (right figure).
Figure 12 shows the preparation of the implant device of the present invention with a dental implant having a periodontal membrane cell sheet wrapped around (top figure), and the schematic diagram of the implant procedure (bottom figure).
Figure 13 shows the micro CT images showing the change in tissue over time after transplanting an implant body with the dental implant device of the present invention. At about 4 weeks after transplantation, periodontal space is recognized between the implant superficial layer and the surrounding alveolar bone.
Figure 14 shows the tissue image (HE staining image) around the implant body at about 4 weeks after transplanting an implant body with the dental implant device of the present invention. Tissue structure equivalent to the periodontium of a natural tooth such as the cementum, the periodontal membrane, and the alveolar bone is recognized on the superficial layer of the implant body, showing that it is surviving on the jaw bone.
Figure 15 shows the tissue image (azan staining image) around the implant body at about 4 weeks after transplanting an implant body with the dental implant device of the present invention. The presence of distinctly running periodontal membrane fibers that connect the superficial layer of the implant body with the surrounding alveolar bone is shown.
Figure 16 shows design examples 1 - 3 of the implant fixture employed in the dental implant device of the present invention.
Figure 17 shows design example 4 of the implant fixture employed in the dental implant device of the present invention.
Figure 18 shows design example 5 of the implant fixture employed in the dental implant device of the present invention.
Figure 19 shows the CT scan results of an adult beagle dog mandible.
Figure 20 shows the implant used in Example 3.
Figure 21 shows the correspondence between putative human clinical procedure and the experimental procedure in Example 3.
Figure 22 shows the dental implant device (dental implant and implant fixture) used in Example 3.
Figure 23 shows the transplantation procedure of a dental implant device in Example 3.
Figure 24 shows the tissue images of observing tissue regeneration over time and making comparison in the experimental group having a dental implant device transplanted to an extraction socket with periodontal membrane remaining and the control group having a dental implant device transplanted to an extraction socket with periodontal membrane removed.
Figure 25 shows the result of comparing regenerated tissue in the experimental group having a dental implant device transplanted to an extraction socket with periodontal membrane remaining and the control group having a dental implant device transplanted to an extraction socket with periodontal membrane removed.
Figure 26 shows the tissue image around the implant in the experimental group having a dental implant device transplanted to an extraction socket with periodontal membrane remaining.
Figure 27 shows the tissue image around the implant in the control group having a dental implant device transplanted to an extraction socket with periodontal membrane removed.
Figure 28 shows the tissue image of the periodontal membrane tissue (periodontal membrane that was present from before the experiment) of a natural tooth of a test animal.

### Description of Embodiments

An "implant" is generally an appliance used in a human or an animal for a medical purpose for burying in a living body. A "dental implant" as used herein in particular refers to a dental artificial tooth that may be an alternative to a lost tooth when transplanted to a subject.

The dental implant device according to the present invention comprises a dental implant body (may be simply referred to as an implant body herein) and an implant fixture. Said implant fixture comprises a portion to be fixed to said implant body and a portion to be fixed to a tooth adjacent to the transplantation site of said implant body in a subject, and is configured so that mechanical stimulation from the tooth adjacent to said transplantation site is transmissible to said implant body. Said implant device is placed into the oral cavity of said subject so that said implant body is inserted at said transplantation site while said implant fixture is fixed to said implant body and the tooth adjacent to said transplantation site.

"Mechanical stimulation" as used herein includes, but it not limited to, for example physical stimulation such as vibration, compression, and friction.

When the dental implant device of the present invention (dental implant body and implant fixture) is applied into the oral cavity of a subject by configuring the dental implant body and the implant fixture as above, mechanical stimulation (such as vibration and compression) from a tooth adjacent to the transplantation site is transmitted to the implant body, and formation of the periodontium (in particular the periodontal membrane tissue) at the implant transplantation site is promoted. The importance of external mechanical stimulation for the formation of the periodontal membrane tissue is also shown in e.g. WO/2011/125425.

A "periodontium" as used herein refers to a tissue mainly formed on the outer layer of a tooth composed of the cementum, the periodontal membrane, the alveolar bone, and the gingiva. The periodontium formed at the transplantation site by transplantation of the implant device of the present invention is in particular the cementum, the periodontal membrane, and the alveolar bone. The cementum and the periodontal membrane formed after transplantation of the implant device of the present invention forms the periodontium by being ligated with the recipient's alveolar bone or gingiva etc.

The cementum, the periodontal membrane, and the alveolar bone can be morphologically easily identified by for example tissue staining. As the staining method, for example ordinary hematoxylin/eosin (HE) staining can be employed. Upon tissue staining, those skilled in the art can perform HE staining by for example going through the steps of fixing the sample with 4% paraformaldehyde (PFA), decalcifying with 10% ethylenediaminetetraacetic acid (EDTA), paraffin embedding, and then producing serial sections having a thickness of 10 micrometers, etc. Moreover, in addition to the above exemplification, it is possible for those skilled in the art to perform tissue staining according to a common method and carry out histological evaluation.

The dental implant device of the present invention can be applied to a tooth loss site in the oral cavity, preferably it can be applied to an extraction socket. Note that an extraction socket as used herein means a site of missing tissue set in the gingiva by tooth extraction etc., and is not limited to those of a particular shape. When applying the implant device of the present invention to an extraction socket, for example it is preferred that it is applied within seven days after tooth extraction. Mesenchymal lineage stem cells or progenitor cells are present in the granulation tissue of the extraction socket up until approximately Day 7 after tooth extraction, and it is known that these stem cells or progenitor cells may differentiate into various tissues (such as the periodontal membrane tissue, the alveolar bone, and the cementum) (e.g. Nakajima R. et al., Mesenchymal stem/progenitor cell isolation from extraction sockets. Journal of Dental Research; 93(11): 1133-1140, 2014).

Moreover, when the dental implant device of the present invention is applied to the extraction socket, it is preferred that said implant body is inserted in said extraction socket so as not to contact the bottom of said extraction socket and is fixed by said implant fixture, to thereby maintain a state where said implant body does not contact the bottom of said extraction socket. By inserting the implant body in the extraction socket to an extent that it does not contact the bottom of the extraction socket, and fixing and maintaining it, the periodontium can be formed throughout the entire insertion site of the implant body in the extraction socket. When the dental implant device of the present invention is applied as above, the distance between the implant body inserted in the extraction socket and the bottom of the extraction socket may be maintained at for example 0.1 - 5.0 mm, preferably 0.3 - 4.0 mm, more preferably 0.5 - 3.0 mm, further preferably 0.7 - 2.5 mm, and most preferably 1.0 - 2.0 mm. When the distance between the implant body inserted in the extraction socket and the bottom of the extraction socket is too close (such as 0.1 mm or less), or when the distance is too far (such as 5.0 mm or more), formation of appropriate periodontium may not be carried out. Note that transplantation of the implant in an aspect such as above is sometimes referred to herein as "suspension implant transplantation."

As the material of the implant body used in the present invention, those conventionally employed as implant body material can be used as long as it is not harmful to the living body, has affinity to the living body, and is a material of high strength that may withstand occlusion. Examples of an implant material can include a metal material, a metal alloy material, a plastic material, a ceramic material, a composite material, a bone alternative material, and the like.

In the present invention, examples of the metal and metal alloy that may be used as the implant body can include titanium, steel, iron, alloy steel, iron alloy, a titanium alloy, a CoCr alloy, silver, copper, calcium, magnesium, zinc, and the like.

In the present invention, examples of the plastic material that may be used as the implant body include polymers such as polyethylene, polypropylene, polytetrafluoroethylene, polyethylene terephthalate, polyamides, polyurethanes, polysiloxanes, polysiloxane elastomers, polyether ether ketones, polysulphones, polysaccharides, and polylactides.

In the present invention, examples of the ceramic material that may be used as the implant body can include oxides or nitrides such as aluminum oxide, zirconium oxide, titanium oxide, and silicon oxide, for example calcium phosphates such as hydroxyapatite, glass and glass ceramics, preferably glass and glass ceramics that dissolve or degrade under physiologic conditions, and the like.

In terms of biocompatibility or mechanical biocompatibility, it is more preferred that titanium or a titanium alloy is employed as the material used in the implant body of the present invention. Moreover, bone alternative materials can include for example an autologous tooth, a tooth obtained from an individual of the same species, or a tooth obtained from an individual of a different species.

The shape or size of the implant body used in the present invention can be appropriately designed by those skilled in the art to adapt to the environment of the transplantation destination (such as the size of the missing tooth site or the relationship with an adjacent tooth).

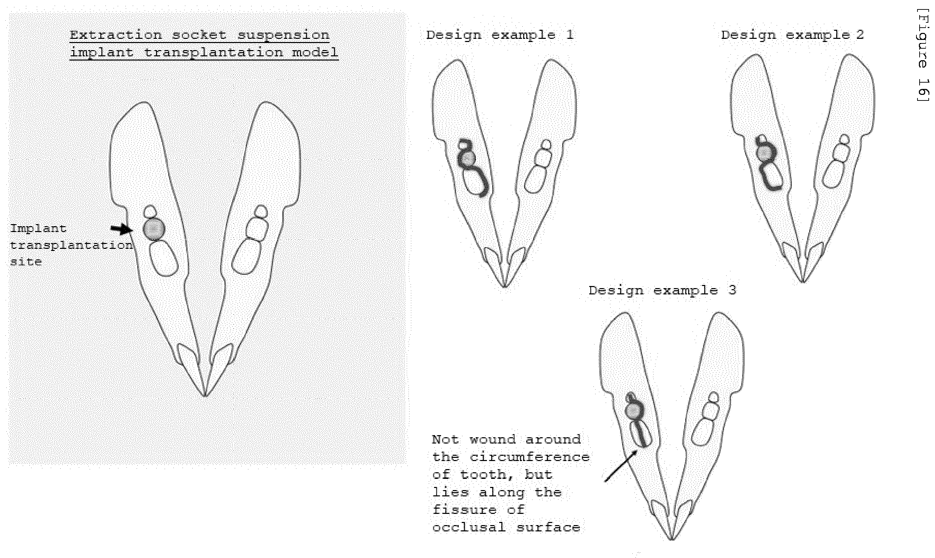

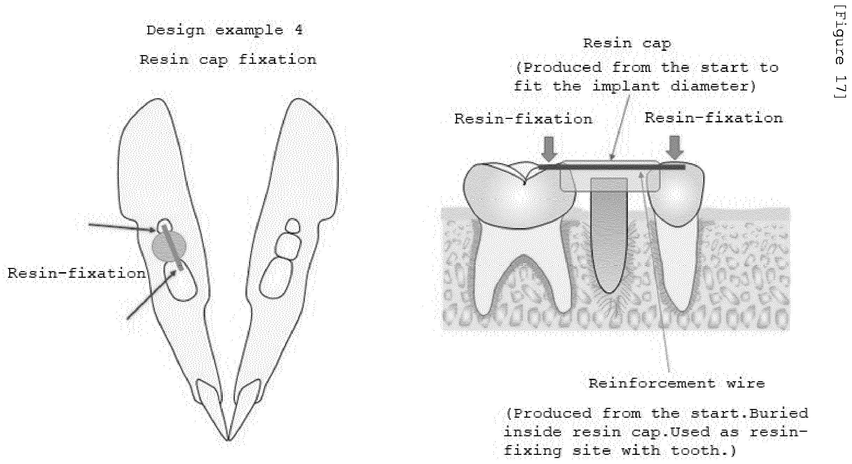

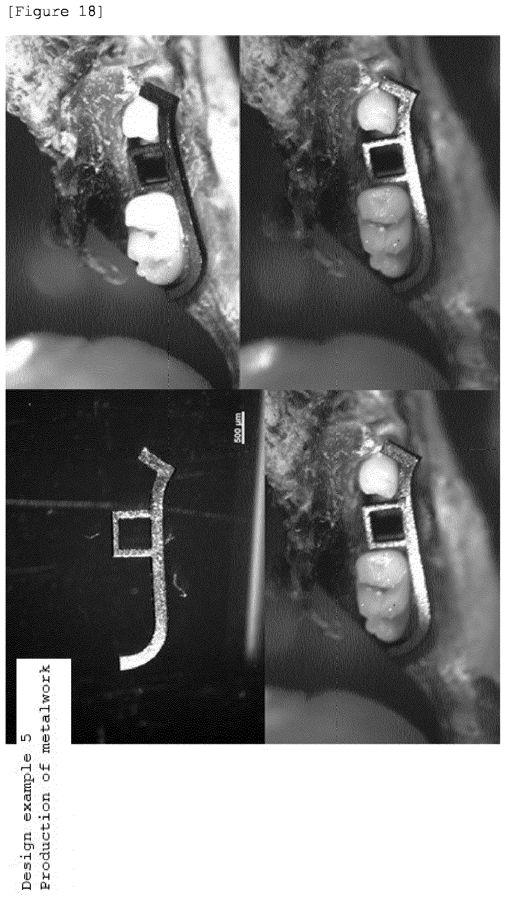

The implant fixture used in the present invention can be freely designed by those skilled in the art without being limited by its shape, structure, and material, as long as it comprises a portion to be fixed to the implant body and a portion to be fixed to a tooth adjacent to the transplantation site of said implant body in a subject, and is configured so that mechanical stimulation from the tooth adjacent to said transplantation site is transmissible to said implant body. For example, the portion to be fixed to the implant body in the implant fixture used in the present invention may be of a design that allows interposing of the implant body such as in design examples 1 - 3 of Figure 16, or may be of a design that allows fixing with resin or cement etc. such as in design example 4 of Figure 17, or may be designed in circular form such as in design example 5 of Figure 18. Similarly, the portion to be fixed to a tooth adjacent to the transplantation site of said implant body in a subject in the implant fixture used in the present invention may be of a design that allows interposing of the adjacent tooth such as in design examples 1 - 3 of Figure 16 or design example 5 of Figure 18, or may be of a design that allows fixing with resin or cement etc. such as in design example 4 of Figure 17. Note that the dental implant device of the present invention does not need to be designed to have the implant body and the implant fixture in directly contact, and for example may be designed as a configuration where the implant body and the implant fixture are indirectly connected via resin or cement etc.

Moreover, the implant fixture used in the present invention may be fixed to adjacent teeth on both sides of the transplantation site of the implant body, or may be fixed only to an adjacent tooth on one side. Those skilled in the art can design an implant fixture in various aspects based on the design examples disclosed herein as well as technical common sense.

The material of the implant fixture used in the present invention is not limited, and is preferably a material that has both rigidity and toughness in order to make mechanical stimulation from a tooth adjacent to the transplantation site of the implant body transmissible to said implant body. For example, the material of the implant fixture used in the present invention is preferably selected from the group consisting of a metal material, a ceramic material, a polymeric material, a hard fiber material, and ebonite.

Examples of the metal employed as the material of the implant fixture in the present invention can include titanium, steel, iron, an alloy steel, an iron alloy, a titanium alloy, a CoCr alloy, silver, copper, calcium, magnesium, zinc, and the like.

Examples of the ceramic material employed as the material of the implant fixture in the present invention include oxides or nitrides such as aluminum oxide, zirconium oxide, titanium oxide, and silicon oxide, for example calcium phosphates such as hydroxyapatite, glass and glass ceramics, preferably glass and glass ceramics that dissolve or degrade under physiologic conditions, and the like.

Examples of the polymeric material employed as the material of the implant fixture in the present invention can include plastic materials (e.g. polymers such as polyethylene, polypropylene, polytetrafluoroethylene, polyethylene terephthalate, polyamides, polyurethanes, polysiloxanes, polysiloxane elastomers, polyether ether ketones, polysulphones, polysaccharides, and polylactides), synthetic resins, resins, and the like.

Examples of the hard fiber material employed as the material of the implant fixture in the present invention can include materials originating from carbon fiber, glass fiber, boron fiber, aramid fiber, polyethylene fiber, Zylon™, and the like.

The ebonite (or hard rubber) employed as the material of the implant fixture in the present invention may be a natural rubber or a synthetic rubber as long as it is a rubber that has the necessary rigidity and toughness, and those skilled in the art can appropriately make the selection.

Moreover, in one embodiment of the present invention, the dental implant body have a coating layer of a surface coating agent formed on its surface. A "surface coating agent" as used herein refers to those that may be used in forming a scaffolding when adhering cells to the implant. The coating layer of a surface coating agent formed on the implant surface can improve adherence of cells to the implant.

Examples of the surface coating agent used for the present invention can include gel materials such as hydroxyapatite, α-TCP (tricalcium phosphate), β-TCP, or collagen. In particular, hydroxyapatite has the biological activity to promote osteogenesis, and can promote the formation of cementum around the implant body after implant transplantation or promote the survival of the implant on bones. In these regards, hydroxyapatite is preferred as the surface coating agent used for the present invention.

The surface coating agent used for the present invention can be coated over all or a part of the surface of the implant body, which is the top of the surface that will be surrounded by the recipient's tissue (such as extraction socket tissue) at the time of implant transplantation. Moreover, the coating layer of a surface coating agent may be formed so as to be interpositioned between the implant and the cell mass to be placed on the implant.

The top of the surface of the implant body that will be surrounded by the recipient's tissue (such as extraction socket tissue) at the time of implant transplantation refers to the portion that will be buried into the missing tooth site of the recipient immediately after implant transplantation. This portion is the portion that will ligate with the periodontium of the recipient in the future.

Note that those having an effect similar to a surface coating agent may be used, such as using for example hydroxyapatite as the material of the implant body of the present invention. In such a case, application of a surface coating agent to the implant body is unnecessary, but another surface coating agent may further be applied.

The coating of a surface coating agent to the implant body can be carried out by a method well-known to those skilled in the art. For example, when hydroxyapatite is coated on the implant body, this can be carried out by vapor deposition, plasma spraying method, and the like. The thickness of the surface coating agent layer or the extent of coating of the surface coating agent can be appropriately set by those skilled in the art depending on the implant body subject to coating or the state of the transplantation destination and the like. For example, in one embodiment of the present invention, the thickness of the coating layer can be 1 µm - 2 µm. Moreover, in addition to forming a coating layer of a surface coating agent on the implant body surface as above, a commercially available implant body having a surface coating agent such as hydroxyapatite already coated thereon may be used in the present invention.

In one embodiment of the present invention, a periodontal membrane tissue-derived or tooth germ tissue-derived cell mass may be placed on all or a portion of the surface of the implant body. It has been disclosed for example in WO2013/115128 that functional periodontium may be formed on the implant surface after implant transplantation by placing a tooth germ tissue-derived or periodontal membrane tissue-derived cell mass on the dental implant surface for subjecting to transplantation, and a synergistic effect can be obtained by combining with the implant device of the present invention.

A "cell mass" as used herein refers to the entirety of the originating tissue or a portion thereof that at least partially maintains the functional bond between cells that form the tissue. In terms of ease of placement to the implant body, it is preferred that the cell mass is a cell sheet. A "cell sheet" as used herein refers to that where multiple cells are adhered to one another in sheet form.

The method for preparing the cell sheet employed in the present invention (such as a periodontal membrane tissue-derived cell sheet) is not limited, and preparation can be carried out with a method well-known to those skilled in the art. For example, by dispersing a periodontal membrane tissue harvested from a living body, and then culturing in a cell culture dish filled with culture medium (or a dish coated with a scaffolding such as collagen gel or Matrigel™), a periodontal membrane tissue-derived cell sheet can be prepared. The periodontal membrane cell to be the cell sheet material for example may be harvested from the periodontal membrane of an extracted tooth (such as an extracted wisdom tooth), or the periodontal membrane tissue of a normal tooth may be partially excised without tooth extraction and employed. Moreover, as another method, a cell sheet may be prepared with periodontal membrane cells differentiation-induced from ES cells, iPS cells, various tissue stem cells, and the like.

A "tooth germ" as used herein is an early embryo of a tooth destined to be a tooth in the future, and refers to those in the stages of the Bud stage, the Cap stage to the Bell stage generally employed in the developmental stages of teeth, in particular a tissue in which accumulation of dentin and enamel characteristic as hard tissue of teeth is not recognized.

In the present invention, for example, tooth germ tissues in the Cap stage, early Bell stage, and late Bell stage can be used. These tooth germs in the Cap stage, early Bell stage, and late Bell stage are preferred in that they have high differentiation potential into functional periodontium when transplanted together with the implant body. In particular, it is more preferred to use tooth germ in the early Bell stage. A cell mass derived from tooth germ in the early Bell stage is preferred in that is can further promote the formation of functional periodontium accompanied by the formation of the cementum. In case of a mouse, fetal age Days 13 - 15 correspond to the Cap stage, fetal age Days 16 - 18 correspond to the early Bell stage, and fetal age Day 19 - after birth correspond to the late Bell stage.

Moreover, in the present invention, a "regenerated tooth embryo" artificially formed by cell culture technology can also be used. A cell mass can also be harvested at the preferred developmental stage when using a regenerated tooth embryo. The regenerated tooth embryo used in the present invention may be those produced by any method, and for example can by produced by a method comprising a step of placing in close contact a first cell aggregate composed substantially of mesenchymal lineage cells and a second cell aggregate composed substantially of epithelial cells and a step of culturing the first and second cell aggregates inside a support carrier.

Methods for manufacturing a regenerated tooth embryo are described in for example International Publication No. 2006/129672, Japanese Published Unexamined Patent Application Publication No. 2008-29756, Japanese Published Unexamined Patent Application Publication No. 2008-206500, Japanese Published Unexamined Patent Application Publication No. 2008-200033, Japanese Published Unexamined Patent Application Publication No. 2008-29757, International Publication No. 2011/056007, and International Publication No. 2011/056008, the disclosures of each of these literatures incorporated herein by reference in their entirety.

Tooth germ and other tissues can be harvested from the jaw bone or the periodontium etc. of mammal primates (such as humans and monkeys), ungulates (such as pigs, cows, and horses), small mammal rodents (such as mice, rats, and rabbits), as well as various animals such as dog and cats. Ordinarily, for the harvesting of the tooth germ and the tissue and the separation of the tissue from a tooth germ, conditions employed for tissue harvesting may be applied without modification, and it may be carried out by removing under sterile condition and storing in an appropriate preservation solution (note that it may not need to be a strict sterile condition). While the tooth germ from the third molar (the so-called wisdom tooth) as well as fetal tooth germ can be listed as the human tooth germ that may be used in the present invention, in terms of utilizing autologous tissue, it is preferred to employ the tooth germ from the wisdom tooth.

Examples of a tooth germ tissue-derived cell mass that may be used in the present invention includes the tooth germ mesenchymal tissue and the dental follicle tissue. The tooth germ mesenchymal tissue exists in a tooth germ of the Bud stage, the Cap stage, or the early Bell stage. Moreover, the dental follicle tissue exists in a tooth germ of the early Bell stage and the late Bell stage. Moreover, the periodontal membrane tissue used in the present invention can be harvested from a complete tooth. Ordinarily, for the separation of tissues such as the tooth germ mesenchymal tissue and the dental follicle tissue from a tooth germ tissue and the separation of the periodontal membrane tissue from a complete tooth, conditions employed for tissue harvesting may be applied without modification, and it may be carried out by removing under sterile condition and storing in an appropriate preservation solution. In doing so, an enzyme may be employed for carrying out the separation easily. Examples of an enzyme can include dispase, collagenase, and trypsin.

Moreover, the tissue resected from a living body can be physically cut into several masses of cells to use as the cell mass. In doing so, it is preferred that the cell mass is cut so that the functional bond between cells when they had formed a tissue is partially retained. In particular, it is more preferred that the cell mass after cutting retains the shape that enables discrimination between the exterior and the interior of the tissue. In this way, the cell mass used in the present invention enables the formation of a periodontium having normal function when transplanted together with the implant body by partially possessing the functional bond between cells when they had formed a tissue. Note that the shape of the cell mass cut apart is not particularly limited as long as it has a shape that allows easy placement onto the surface of the implant body, and for example can be cut apart long and thin as in a strip form. Ordinarily, for the cutting apart of such a cell mass, conditions employed for tissue harvesting may be applied without modification.

The method for placing the cell masses onto the surface of the implant body is not particularly limited. For example, cell masses cut apart in strip form can be pasted onto the surface of the implant body so that the cell masses do not overlap each other. Alternatively, they can also be placed by wrapping onto the surface of the implant body so that the cell masses do not overlap each other. In doing so, adhesiveness is improved if the implant body having the cell masses pasted is dried a little in air. Moreover, when there is a coating layer of a surface coating agent on the surface of the implant body, the cell masses can be placed on the further surface of the coating layer of a surface coating agent.

In one aspect of the present invention, the position for placing the cell mass onto the surface of the implant body can be at all or a part of the surface that will be surrounded by the recipient's tissue (such as extraction socket tissue) after transplantation of the implant body. Moreover, it is preferred that they are placed to the extent that the implant body is implanted into the recipient's tissue. Moreover, it is preferred that they are placed so that the side that had formed the interior of the tissue in the cell mass is in contact with the implant surface. By placing in this way, the side that had formed the exterior of the tissue will face the alveolar bone of the recipient.

Another aspect of the present invention relates to a method of transplanting a dental implant device to a subject with the dental implant device of the present invention. Animals to which the method of transplanting a dental implant according to the present invention can be applied are not limited, and for example it can be applied to mammals including humans, cows, horses, pigs, dogs, cats, mice, and the like. Note that when a periodontal membrane tissue-derived or tooth germ tissue-derived cell mass is placed on an implant body for transplantation, it is preferred that the recipient is the same species as the animal from which the said tooth germ tissue or the periodontal membrane tissue is resected, and it is further preferred that it is the same individual as the individual from which the tooth germ tissue or the periodontal membrane tissue was resected.

The implant transplantation site can be morphologically easily observed by those skilled in the art by visual confirmation or CT imaging. CT images or CT section images can also be easily scanned by those skilled in the art by employing well-known instruments. For a CT scan, for example a 3D micro X-ray CT for laboratory animals R_mCT (Rigaku Corporation) can be employed, and it can be run for example under a condition of 90 kV, 150 mA, and a tomography thickness of 10 mm. Moreover, those skilled in the art will be able to perform image construction and analysis etc. after CT scan with an appropriate image analysis software. Examples of image analysis softwares that can be employed are for example image filing software for small animals i-VIEW Type R and high-resolution 3D/4D image analysis software Imaris (Bitplane). Moreover, in addition to the exemplifications above, those skilled in the art will be able to perform CT scan and analysis by setting appropriate conditions with a similar instrument and utilizing a similar image analysis software.

According to the implant device of the present invention, functional periodontium can be formed around the implant body after transplantation to a recipient. In the present invention, functional periodontium refers to those (i) possessing a functional cementum and a functional periodontal membrane, or (ii) possessing functional nerve fibers, and preferably refers to those possessing both characteristics of (i) and (ii).

In other words, functional periodontium can be evaluated for example by whether or not it possesses a functional cementum and a functional periodontal membrane. For example, a functional cementum and a functional periodontal membrane can be evaluated by verifying whether it possesses a layered structure equivalent to the periodontium of a natural tooth when performing histological analysis such as by HE staining and azan staining. Here, the periodontal membrane of a natural tooth ordinarily has periodontal membrane fibers that run vertical against the longitudinal direction of the tooth root. This periodontal membrane has an important role particularly in supporting the occlusal force of teeth. Accordingly, by analyzing whether periodontal membrane fibers that run vertical against the longitudinal direction of the implant similarly to natural teeth are formed, in particular the function of the periodontal membrane can be evaluated. In addition to the above method, for analyzing the morphology of the periodontium or for analyzing the run of the periodontal membrane, the morphology thereof can also be observed by for example a scanning electron microscope or a transmission electron microscope. Moreover, in order to verify the three-layer structure of hard tissue-fibrous tissue-hard tissue in the periodontium, for example the layered structure of the periodontium can be verified by analyzing the elemental distribution with an X-ray microanalyzer.

As an alternative method, the function of bone remodeling against corrective force loading can be evaluated. Alternatively, evaluation can be performed by analyzing whether or not the implant after transplantation is movable by the corrective force loading. For evaluation of bone remodeling, evaluation can be performed for example by analyzing the expression of an osteogenic marker and/or a bone resorption marker after corrective force loading.

Moreover, functional periodontium can be evaluated for example by whether or not it possesses functional nerve fibers. A functional nerve fiber refers to a nerve fiber that can transmit stimulation to the central nervous system upon stimulation etc. to the periodontium. Whether or not the nerve fiber is functional can be evaluated for example by applying stimulation by corrective force loading to the periodontium of an evaluation subject, and then analyzing the expression of c-fos in the trigeminal tract nucleus.

Note that the terms used herein are employed for describing particular embodiments, and do not intend to limit the invention.

Moreover, the term "comprising" as used herein, unless the content clearly indicates to be understood otherwise, intends the presence of the described items (such as components, steps, elements, and numbers), and does not exclude the presence of other items (such as components, steps, elements, and numbers).

Unless otherwise defined, all terms used herein (including technical and scientific terms) have the same meanings as those broadly recognized by those skilled in the art of the technology to which the present invention belongs. The terms used herein, unless explicitly defined otherwise, are to be construed as having meanings consistent with the meanings herein and in related technical fields, and shall not be construed as having idealized or excessively formal meanings.

The embodiments of the present invention may be described with reference to schematic diagrams. In such a case, they may be exaggerated in presentation in order to allow clear description.

Terms such as first and second are employed to express various elements, and it should be recognized that these elements are not to be limited by these terms. These terms are employed solely for the purpose of discriminating one element from another, and it is for example possible to describe a first element as a second element, and similarly, to describe a second element as a first element without departing from the scope of the present invention.

Any and all numeric values employed herein for indicating numeric value range and the like, unless explicitly indicated, is construed as encompassing the meaning of the term "about." For example, "10 folds," unless explicitly indicated, is understood as meaning "about 10 folds."

All of the disclosures of the literatures cited herein should be deemed as cited herein, and those skilled in the art shall cite and recognize the related disclosed contents in these prior art literatures as a part of the present specification according to the context herein and without departing from the spirit and scope of the present invention.

The present invention will now be described in further detail with reference to Examples. However, the present invention can be embodied by various aspects, and shall not be construed as being limited to the Examples described herein.

### Examples

### Example 1: Suspension implant transplantation model

In recent dental research, extraction socket-derived stem cells having cellular nature different from that of bone marrow-derived stem cells have been identified in the granulation tissue harvested from the extraction socket immediately after tooth extraction, and it has been shown that these cells have not only the nature as stem cells possessing multipotency but also enables periodontium formation (the cementum, the periodontal membrane, and the alveolar bone) (Nakaj ima R. et al., Journal of Dental Research; 93(11): 1133-1140, 2014.)

In this Example, the following experiments were carried out in order to demonstrate a next-generation implant transplantation model that utilizes the periodontal membrane tissue present in the extraction socket (extraction socket stem cells maintained in the tissue).

### (1) Periodontal membrane tissue in the extraction socket

### Experiment method

In order to demonstrate a next-generation implant transplantation model that utilizes the periodontal membrane tissue present in the extraction socket (extraction socket stem cells maintained in the tissue), it is necessary to demonstrate that healthy periodontal membrane tissue sufficiently remains in an extraction socket in a mouse jaw bone. Accordingly, with the mandibular second molar (M2) of a 4 weeks-old C57BL/6 mouse as the target, a 25 G needle (NN-2516R, TERUMO CORPORATION, Tokyo, Japan) was employed to detach the periodontal membrane tissue around the M2 tooth root from the surface of M2, and then the same tooth was extracted (in other words, the periodontal membrane tissue remains on the alveolar bone side even after M2 tooth extraction) . After tooth extraction, micro-CT scans (In vivo Micro X-ray CT System; R_mCT, Rigaku Corporation, Tokyo, Japan) and image data of the extraction socket were evaluated for the condition of the extraction socket with an integrated image processing software (i-VIEW-3DX, J. MORITA CORPORATION, Osaka, Japan). Further, the mandible after tooth extraction was resected, and then fixed in 4% paraformaldehyde (PFA) for 24 hours. The resected tissue was decalcified for 24 hours with an acidic decalcifying solution formulated by Plank-Rychlo, embedded in paraffin according to a conventional method, and then serial sections of 8 µm were produced to perform histological evaluation by hematoxylin/eosin (HE) staining.

### Result

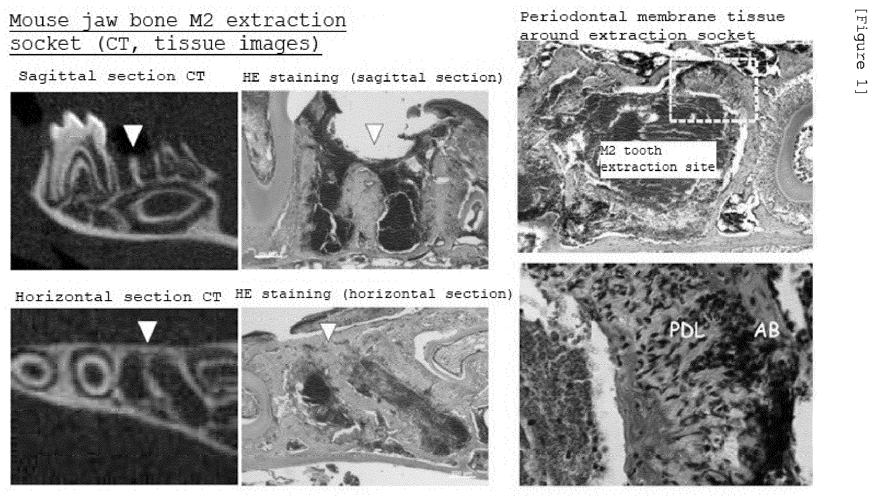

It became clear that the jaw bone after extracting the mandibular second molar (M2) of a 4 weeks-old C57BL/6 mouse is maintained without fracture or damage of the alveolar bone of the adjacent tooth and the septal bone between tooth roots. Further, by observing tissue images (HE staining images) of the jaw bone after tooth extraction, it was shown that the periodontal membrane tissue with distinct run of fibers ligated to the alveolar bone wall around the tooth root remains around almost the whole circumference (Figure 1).

### (2) Suspension fixed implant transplantation model

### (a) Production of hydroxyapatite (HA) implant for mouse

The edge face of pure titanium type I wire (KANEHIRA STEEL CO., LTD., Osaka, Japan) having a diameter of 0.4 mm was rounded by polishing, and the surface was roughened by blast treatment. Subsequently, hydroxyapatite was coated onto the pure titanium surface by flame spraying method at about 3000°C. The pure titanium wire was cut out at a total length of 1.3 mm, and then subjected to vacuum heat treatment and washing. The hydroxyapatite spray coating had a thickness of 20 µm, a Ca/P ratio of 1.66, and a crystallinity of about 60%.

### (b) Production of implant device

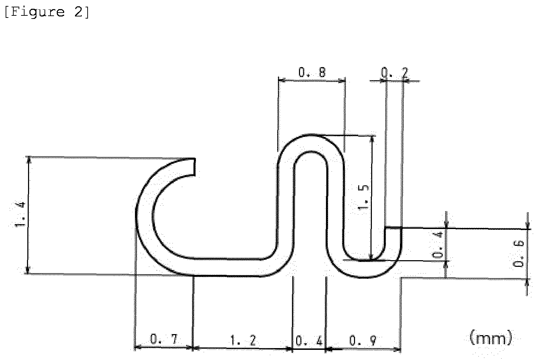

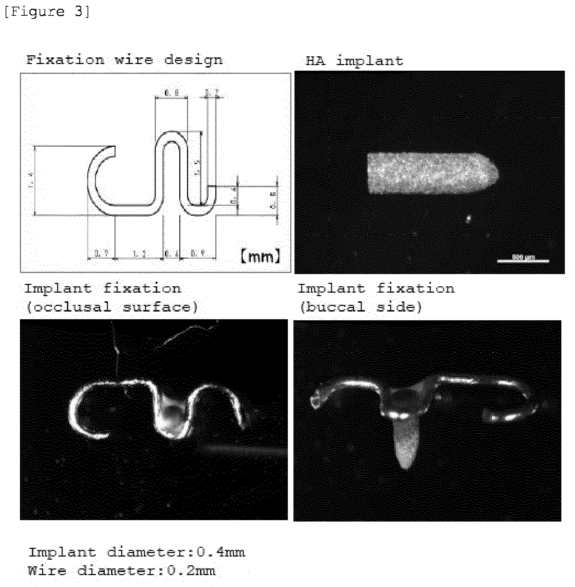

In order to promote formation of periodontal membrane and maturation of the implant, an implant device that can transmit the occlusional load of the animal to the periodontal membrane tissue as mechanical stimulation was developed. An orthodontal wire having a diameter of 0.2 mm was bent with wire pliers, and shaped so that it fits along the right jaw of a 3 weeks-old female C57BL/6 mouse which had the mandibular second molar extracted (Figure 2; implant fixture). To the wire produced, the HA implant produced in item (a) was fixed with a dental resin (Clearfil™ Majesty™ ES Flow, KURARAY NORITAKE DENTAL INC., Tokyo, Japan) (Figure 3).

### (c) Transplantation to mouse

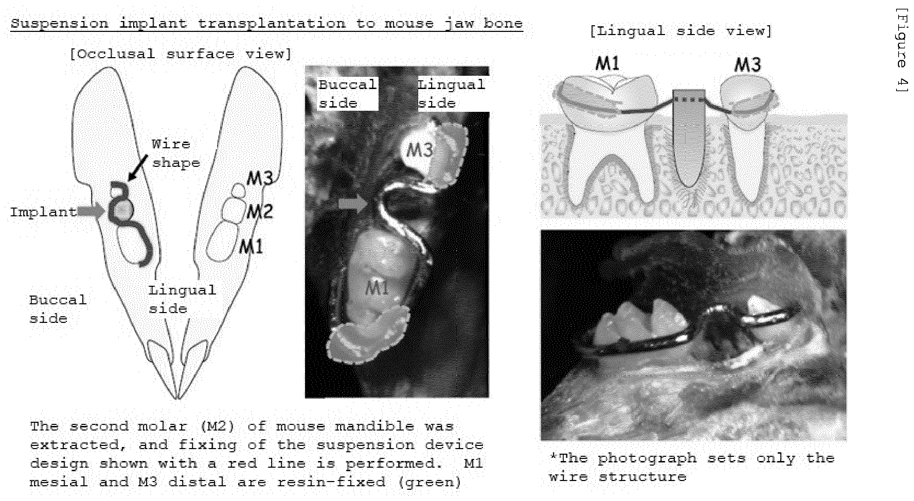

With the mouse mandibular M2 tooth extraction model described above in which tooth extraction is performed leaving the periodontal membrane, implant transplantation by the above device was performed. With the mandibular second molar (M2) of a 4 weeks-old C57BL/6 mouse as the target, a 25 G needle was employed to detach the periodontal membrane tissue around the M2 tooth root from the surface of M2, and then the same tooth was extracted. Subsequently, the septal bone between the tooth roots of M2 was removed with a dental micromotor (Viva-Mate Plus, NSK Nakanishi, Tokyo, Japan) and a dental reamer (MANI, Tochigi, Japan), removing only the septal bone and not damaging the surrounding tissue (the remaining periodontal membrane). Next, the suspension device having the above HA implant fixed was inserted to the M2 extraction socket, and fixed with a resin to the M1 mesial side/M3 distal side which are the adjacent teeth. The length of the implant portion inserted for the suspended implant body was specified at 1.3 mm so as not to contact the socket bottom of the extraction socket (Figure 4).

The mouse which had the implant body transplanted was subjected to micro CT scanning immediately after transplantation and at Days 7, 14, and 21 (In vivo Micro X-ray CT System; R_mCT, Rigaku Corporation, Tokyo, Japan), and image data was evaluated over time with an integrated image processing software (i-VIEW-3DX, J. MORITA CORPORATION, Osaka, Japan) for binding at the implant body and the alveolar bone of the recipient. Moreover, on transplantation Day 30 (4 weeks), the jawbone including the implant body was resected, and the sample treated by the method described above was histologically evaluated (HE, Azan staining) .

### (3) Result

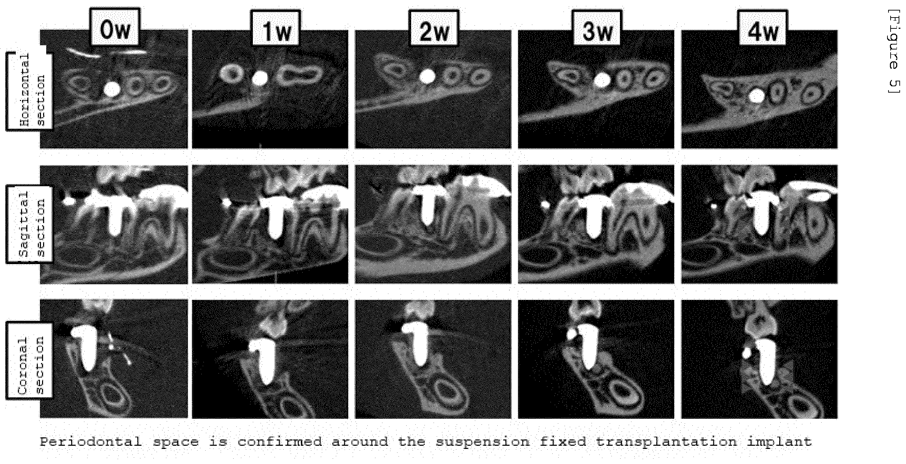

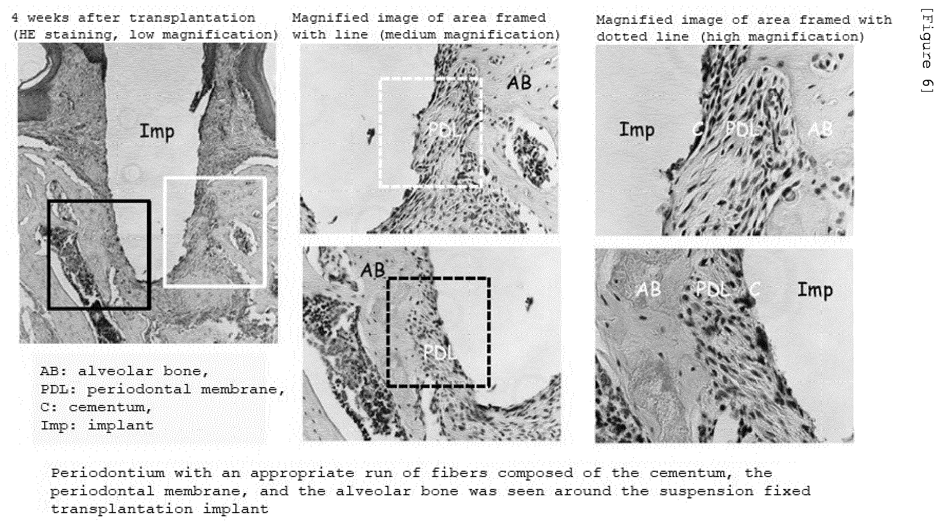

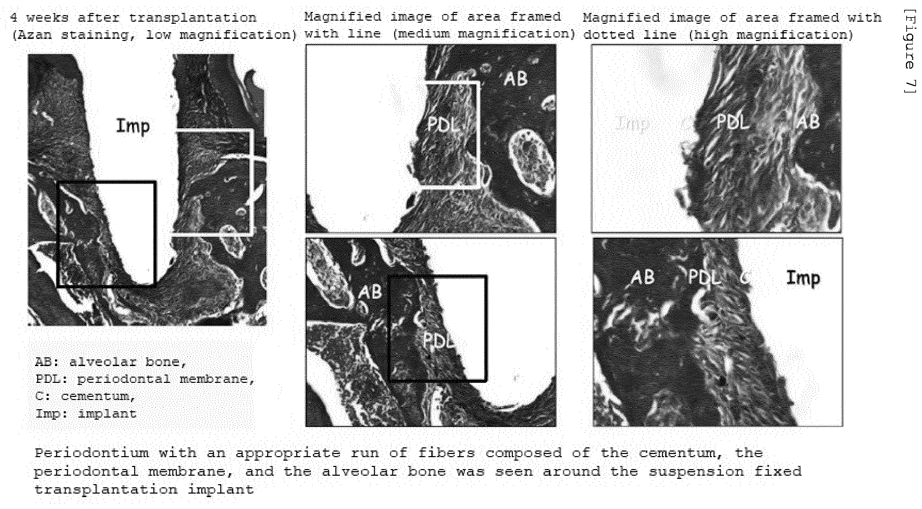

From the micro CT result, in the suspension transplanted implant body, distinct periodontal space was recognized between the implant superficial layer and the surrounding alveolar bone on transplantation Day 30 (Figure 5). Further, in the HE staining image, tissue structure equivalent to the periodontium of a natural tooth such as the cementum, the periodontal membrane, and the alveolar bone was recognized in the implant superficial layer, showing that it is surviving on the jaw bone (Figure 6). Moreover, by azan staining, the presence of distinctly running periodontal membrane fibers that connect the implant superficial layer with the surrounding alveolar bone became clear (Figure 7).

From these, it was shown that by transplanting an implant by the method of the present invention, an implant having normal periodontium could be realized.

It is known that periodontal membrane tissue undergoes maturation of the tissue and optimization of the run of fibers via mechanical stimulation such as occlusional burden with the opposing tooth, and it was shown that decrease in the width of the periodontal membrane tissue or disturbance in the run of fibers occur when the opposing tooth was experimentally extracted to deprive occlusional burden (Kaneko S. et al., J. Periodontal Res. 36, 9-17, 2001.). Moreover, from past research, it is known that mechanical stimulation by sonic wave vibration has an important influence on the width of the periodontal membrane or the run of fibers in periodontium formation (such as WO/2011/125425). In other words, it is thought that also in the present invention, formation of normal periodontium was promoted due to mechanical stimulation from adjacent teeth (M1 and M3) to which the implant was indirectly fixed being transmitted via the implant to the periodontal membrane tissue.

### Example 2: Suspension implant transplantation model employing periodontal membrane cell sheet

In this Example, a model is shown in which a periodontal membrane cell sheet is prepared separately from the suspension implant shown in Example 1, and wrapped on the suspension implant for transplantation.

### (1) Production of implant device

The hydroxyapatite (HA) implant for mouse and the implant device was produced according to the method described in Example 1.

### (2) Production of periodontal membrane cell sheets, wrapping onto implant

### (a) Harvesting and culturing of mouse periodontal membrane cells

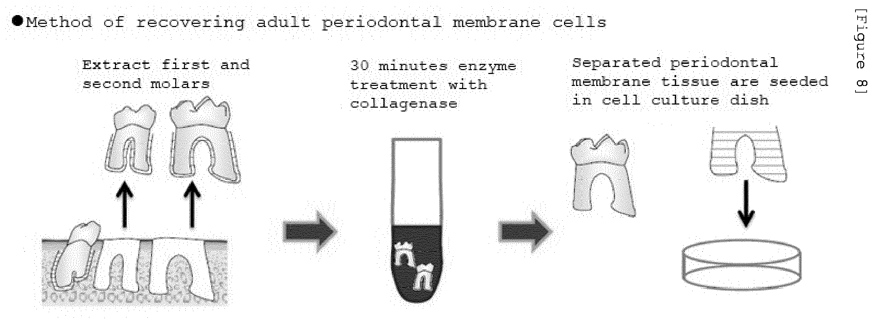

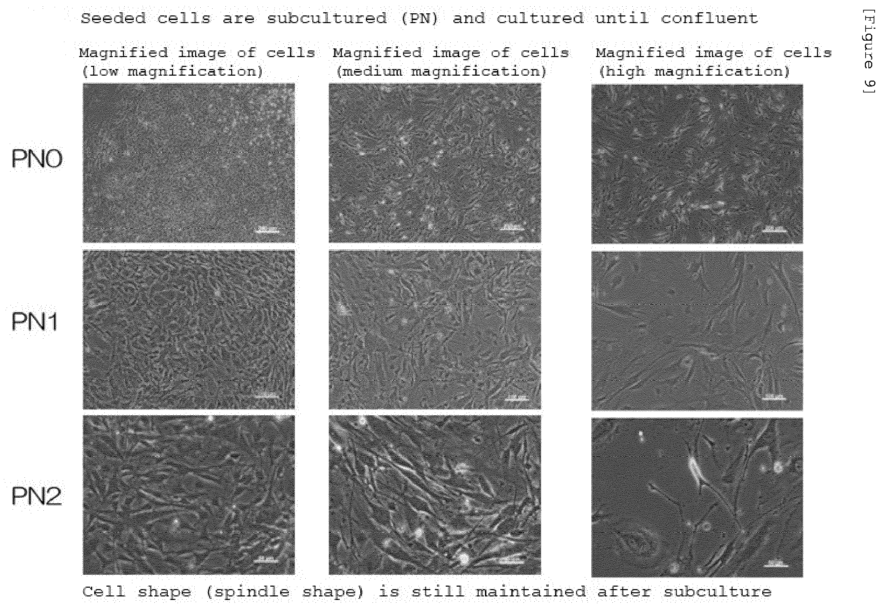

The maxillary and mandibular first and second molars (M1 and M2) of a 4 weeks-old C57BL/6 female mouse were extracted and immersed in collagenase enzyme (Worthington, Lakewood, NJ) . This was treated under a 37°C environment for 30 minutes to detach the periodontal membrane from the molar (Figure 8). The detached periodontal membrane was suspended in glutamine-containing α-MEM (Thermo Fisher Scientific, Inc., Waltham, MA) supplemented with 15% Fetal calf serum (FCS) (Thermo Fisher Scientific, Inc., Waltham, MA), 100 µmol/L L-ascorbic acid-2-phosphoric acid (WAKO, Osaka, Japan), 100 U/ml penicillin, 100 mg/ml streptomycin (Thermo Fisher Scientific, Inc., Waltham, MA), and 100 ng/mL b-FGF (WAKO, Osaka, Japan), and seeded in a dish. The dish with the cells seeded was transferred to an incubator, and cultured at 37°C under a 5% CO₂ environment. During the culture period, medium exchange with the above composition was performed every 2 - 3 days (Figure 9).

### (b) Production of mouse periodontal membrane cell sheets

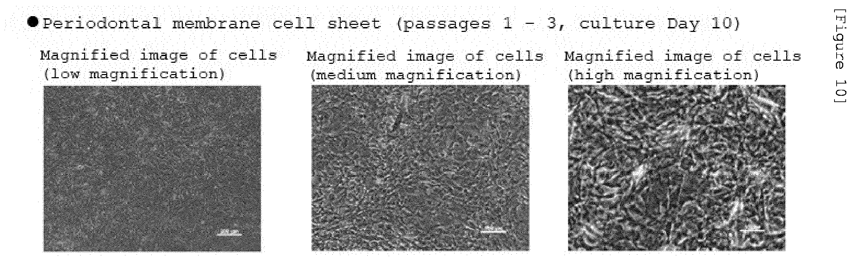

First passage mouse periodontal membrane cells were suspended in glutamine-containing α-MEM supplemented with 15% FCS, 100 µmol/L L-ascorbic acid-2-phosphoric acid, 100 U/ml penicillin, 100 mg/ml streptomycin, and 100 ng/mL b-FGF, and seeded in a temperature responsive cell culture dish (CellSeed Inc., Tokyo, Japan) at a cell density of 1 x 10⁴ cells/cm². The wells were transferred to an incubator, and cultured at 37°C under a 5% CO₂ environment for 10 days. During the culture period, medium exchange with the above composition was performed every 2 - 3 days . A periodontal membrane cell sheet was formed by culturing the periodontal membrane cells in the cell culture dish in sheet form (Figure 10).

### (c) Wrapping of mouse periodontal membrane cell sheets onto implant

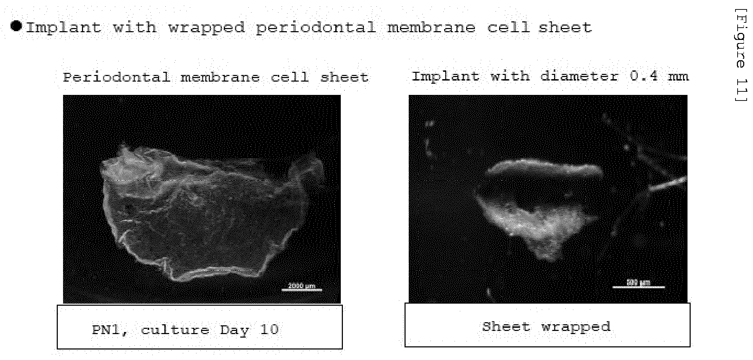

The temperature responsive cell culture dish was transferred into a clean bench at room temperature, and the periodontal membrane cell sheet was detached. The detached and recovered periodontal membrane cell sheet was worked with a 25 G needle (TERUMO CORPORATION, Tokyo, Japan) to an appropriate shape that fits along the implant. The worked periodontal membrane cell sheet was closely wrapped around the whole circumference of the HA implant resin-fixed to the wire (Figure 11).

### (3) Transplantation to mouse

With the mandibular second molar (M2) of a 4 weeks-old C57BL/6 mouse as the target, a 25 G needle was employed to detach the periodontal membrane tissue around the M2 tooth root from the surface of M2, and then the same tooth was extracted. Subsequently, the septal bone between the tooth roots of M2 was removed with a dental micromotor (Viva-Mate Plus, NSK Nakanishi, Tokyo, Japan) and a dental reamer (MANI, Tochigi, Japan) . Further, the periodontal membrane tissue was scraped from the whole circumference of the alveolar wall utilizing a dental reamer so as not to leave any remaining periodontal membrane in the extraction socket.

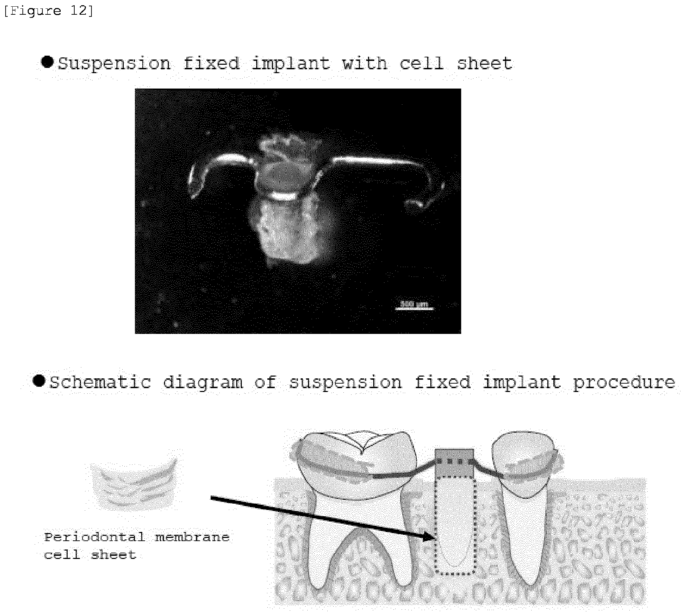

Next, the above implant device having a periodontal membrane cell sheet wrapped around was inserted to the M2 extraction socket, and resin-fixed to the M1 mesial side/M3 distal side which are the adjacent teeth. The length of the implant portion inserted for the suspended implant body was specified at 1.3 mm so as not to contact the socket bottom of the extraction socket (Figure 12).

The mouse which had the implant body transplanted was subjected to micro CT scanning immediately after transplantation and at Days 7, 14, and 21 (In vivo Micro X-ray CT System; R_mCT, Rigaku Corporation, Tokyo, Japan), and image data was evaluated over time with an integrated image processing software (i-VIEW-3DX, J. MORITA CORPORATION, Osaka, Japan) for binding at the implant body and the alveolar bone of the recipient. Moreover, on transplantation Day 30 (4 weeks), the jaw bone including the implant body was resected, and the sample treated by the method described above was histologically evaluated (HE, Azan staining) .

### (4) Result

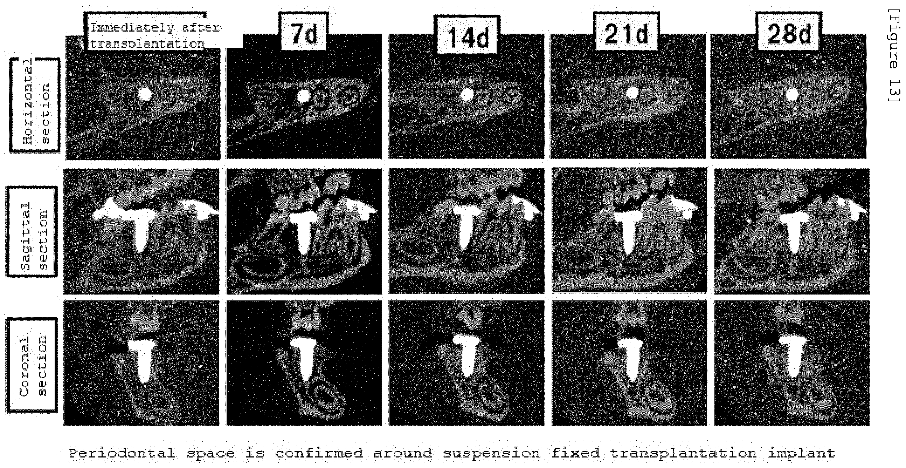

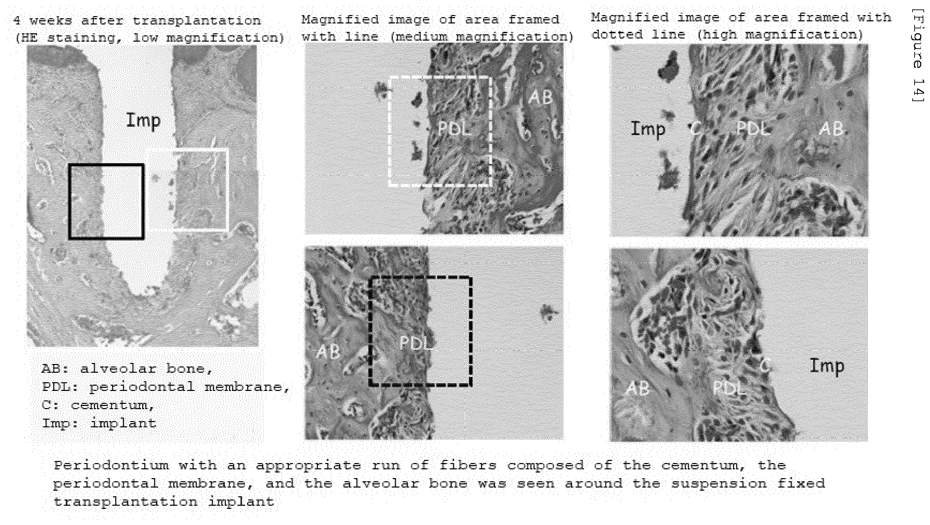

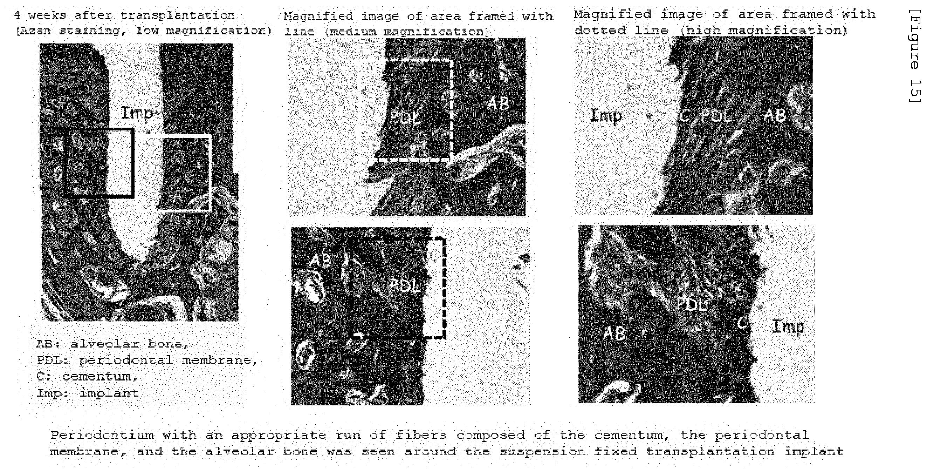

From the micro CT result, in the suspension transplanted implant body, distinct periodontal space was recognized between the implant superficial layer and the surrounding alveolar bone on transplantation Day 28 (Figure 13). Further, in the HE staining image, tissue structure equivalent to the periodontium of a natural tooth such as the cementum, the periodontal membrane, and the alveolar bone was recognized in the implant superficial layer, showing that it is surviving on the jaw bone (Figure 14). Moreover, by azan staining, the presence of distinctly running periodontal membrane fibers that connect the implant superficial layer with the surrounding alveolar bone became clear (Figure 15).

From these, it was shown that even in a case where there is no remaining periodontal membrane present at the transplantation site, by using an artificially manufactured periodontal membrane cell sheet and the suspension implant transplantation model of the present invention in combination, an implant having normal periodontium could be realized.

### Example 3: Suspension implant transplantation model in large animal

In this Example, experiments were carried out with a large animal, i.e. a dog, in order to explore the utility potential of the suspension implant transplantation model of the present invention to a human clinical setting.

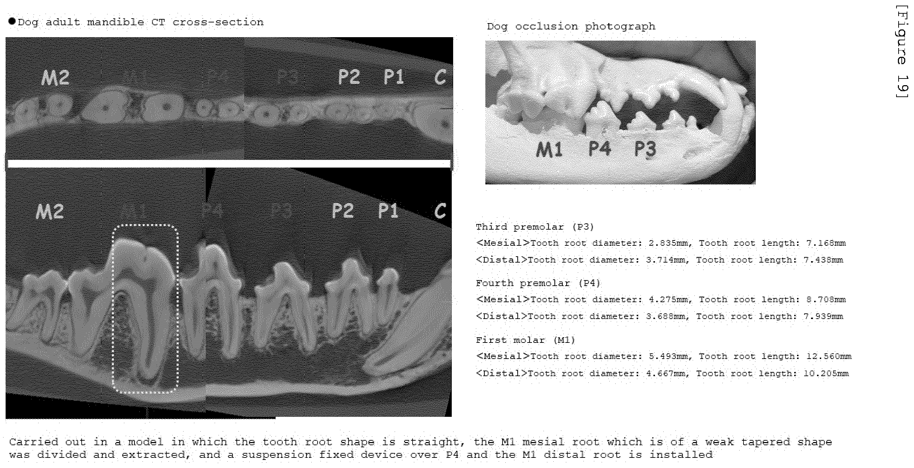

### (1) Selection of next-generation implant transplantation site in dog mandible (Figure 19)

Micro CT scan (In vivo Micro X-ray CT System; R_mCT, Rigaku Corporation, Tokyo, Japan) was performed with the dog jaw bone of the experiment animal, i.e. an adult beagle dog (Toyo-beagle; ORIENTAL YEAST Co., Ltd., Tokyo, Japan), and measurement of the tooth root length and the tooth root width in teeth present in the dog mandible was carried out (image data processing was carried out with an integrated image processing software (i-VIEW-3DX, J. MORITA CORPORATION, Osaka, Japan)). As a result, the tooth root shape of the mesial root of the mandibular first molar (M1) of the adult beagle dog was a straight shape, and together with the tooth root length (12.560 mm) and the tooth root diameter (5.493 mm), it was found to be suitable as the transplantation socket of a biohybrid implant for human clinical use.

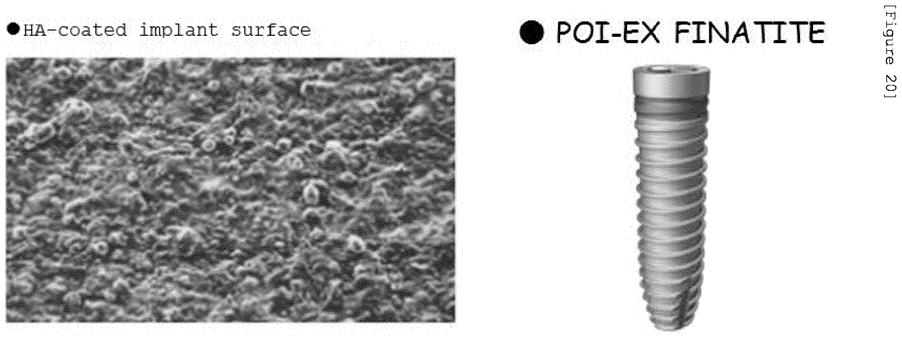

### (2) Selection of implant material used (Figure 20)

In this Example, a hydroxyapatite (HA) coating implant (from KYOCERA Medical Corporation, POI-EX FINATITE tapered type, HAC34-12TP MF: diameter 3.4 mm, length 12 mm) which is an implant that can be inserted into the transplantation socket measured in the above (1) and used in a human dental clinical setting was selected.

The HA surface composition of the said implant is such that the implant surface is roughened by blast treatment, hydroxyapatite is coated by flame spraying method at about 3000°C, and crystallization is carried out by vacuum heat treatment. The thickness of hydroxyapatite is about 20 µm, the Ca/P ratio is 1.66, the crystallinity is 55%, and it has good quality, is stable, and has superior bioaffinity. A post abutment manufactured by the same company (from KYOCERA Medical Corporation, Post AB EX 34-1.0S) is installed at the upper portion of the implant body, and about 2 mm was exposed at the upper portion of the gingiva to render a mechanism that can be ligated with a "suspension fixed device" as described below.

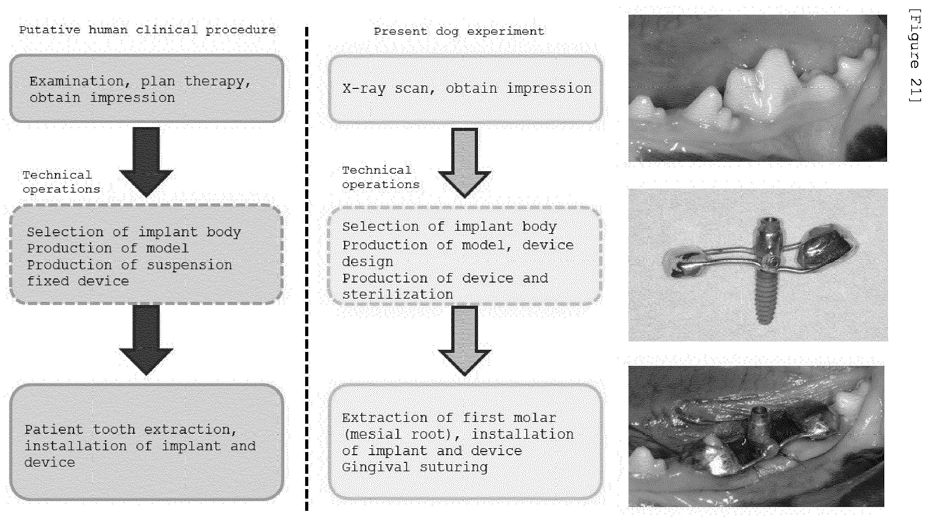

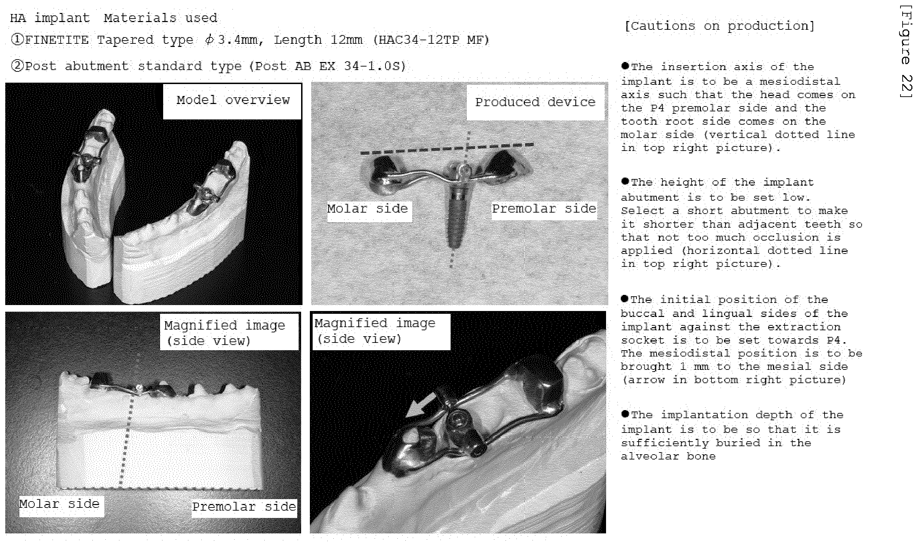

### (3) Production of suspension fixed device (for dog jaw bone) (Figures 21 and 22)

In order to promote formation of periodontal membrane and maturation of the biohybrid implant, a device for implant transplantation that can render the occlusional load intrinsic to the animal as mechanical stimulation was developed.

First, an adult beagle dog was put on general anesthesia (ketamine/xylazine mixed solution: [compound proportion] ketamine (80 mg/kg; Ketalar 500 mg; Daiichi Sankyo Propharma Co., Ltd., Tokyo, Japan) , xylazine (8 mg/kg; Selactar 2% injection; Bayer HealthCare, Tokyo, Japan), and dental calculi in the dog oral cavity was removed. Next, the dog mandibular first molar (M1) that is to be the transplantation socket was scanned with a dental X-ray to obtain information on the tooth root shape and the tooth root length. Subsequently, impression and occlusion registration was obtained for dog maxillary/mandibular dentition with hydrophilic vinyl silicone impression material (Examixfine; GC, Tokyo, Japan) and vinyl silicone impression material for occlusion registration (Exabite III; GC, Tokyo, Japan). Super hard gypsum (New Fujirock; GC, Tokyo, Japan) was poured into the impression material obtained and hardened to produce a high-precision dog dentition model.

The tooth root length and the tooth root width of the M1 mesial tooth root were measured from the X-ray information obtained, a hole with a shape equivalent to the transplantation socket assuming it to be the M1 mesial tooth root was formed on the dog dentition model with a dental micromotor (Viva-Mate Plus, NSK Nakanishi, Tokyo, Japan). In doing so, the tooth crown portion of the M1 distal tooth root was left without grinding. Next, on the dog dentition model having the transplantation socket formed, the fourth premolar (P4) positioned in front of the M1 mesial tooth root and the M1 distal tooth crown portion that was left without grinding in the above were waxed up to render the crown shape, and cast to produce metal crowns compatible to each tooth. The metal crowns produced were produced at a thickness of about 1 mm using 12% gold silver palladium alloy (GC Castwell MC; GC, Tokyo, Japan).

Subsequently, a dental cobalt chromium alloy wire (Sun-cobalt clasp-wire ϕ1.0 mm; Dentsply Sirona K.K., Tokyo, Japan) was employed to render fitting along the shape of the gingival margin from both the buccal and the lingual side so as to connect the metal crowns of the P4 and M1 distal tooth crown portions, and the metal crown and the cobalt chromium alloy wire were soldered with a dental gold solder (IFK Pre-Solder G; ISHIFUKU Metal Industry Co., Ltd., Tokyo, Japan). Finally, a retainer (possesses male part structures on left and right) compatible to the post abutment portion ligated to the upper portion of the HA coating implant in the above (2) was produced/cast by waxing up. Female part structures that receive this retainer were also similarly produced on the cobalt chromium alloy wire side, and fixed at the portion for the implant to be inserted with a dental gold solder. Adjustment of the implantation axis of the implant by about 1 - 10 degrees becomes possible due to this retainer mechanism, allowing to secure an appropriate position so that the inner wall of the transplantation socket and the implant do not come into contact upon transplantation. The final implant position (the height of the post abutment in the oral cavity) was set lower than the occlusion plane, designed so that the implant does not occlude directly with the opposing tooth.

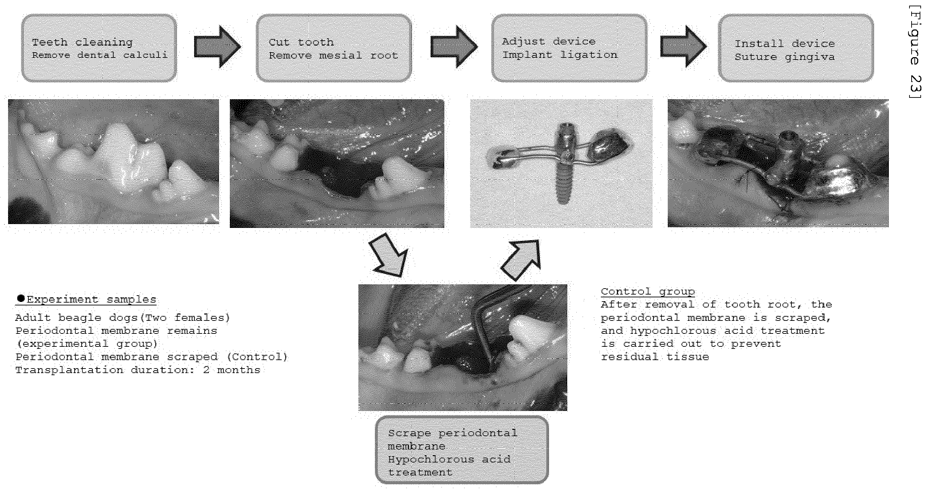

### (4) Suspension fixed implant transplantation method in dog mandible (Figure 23)

An adult beagle dog was put on general anesthesia (ketamine/xylazine mixed solution: [compound proportion] ketamine (80 mg/kg; Ketalar 500 mg; Daiichi Sankyo Propharma Co., Ltd., Tokyo, Japan) , xylazine (8 mg/kg; Selactar 2% injection; Bayer HealthCare, Tokyo, Japan), and dental calculi in the dog oral cavity was removed.

With the dog mandibular first molar (M1) that is to be the transplantation socket as the target, a dental micromotor (Viva-Mate Plus, NSK Nakanishi, Tokyo, Japan) was employed to cut the tooth at the bifurcation between the mesial root and the distal root, and only the M1 mesial root was extracted so as not to damage the periodontal membrane tissue around the tooth root. The suspension fixed device produced in the above (3) was tried in the oral cavity, and fine-tuned so that it would fit in the same site as the dog dentition model. Subsequently, an implant having a post abutment installed was ligated to the suspension fixed device. The implant integrated with the suspension fixed device was then transplanted (inserted) to the transplantation socket (extracted M1 mesial root site), after which the metal crowns of the device (P4, M1 distal site) were fixed to each tooth with dental cement (PANAVIA V5; KURARAY NORITAKE DENTAL INC., Tokyo, Japan).

As the control group in this research, a model in which the periodontal membrane tissue of extraction socket inner wall was mechanically completely removed after tooth extraction of the M1 mesial root was produced, and implant transplantation was similarly carried out. The implantation angle of the implant was verified with a dental X-ray after transplantation and fine-tuned so that the inner wall of the transplantation socket and the implant do not come into contact. The gingiva in the portion in front and behind the transplanted implant was sutured (Softretch 5-0; GC, Tokyo, Japan).

Follow-up after transplantation was performed every 2 weeks by dental X-ray scanning, reflux fixing was performed on Week 9 with a neutral buffered formalin solution (Mildform™ 10N; Wako Pure Chemical Industries, Ltd. Osaka, Japan, the dog mandible was resected, and again immersion fixed in a neutral buffered formalin solution for 48 hours. The sample was embedded in MMA (Methylmethacrylate) resin after trimming the surrounding bone, then undecalcified polished samples with a thickness of 50 micrometers were produced, and subjected to histological evaluation by hematoxylin/eosin (HE) staining.

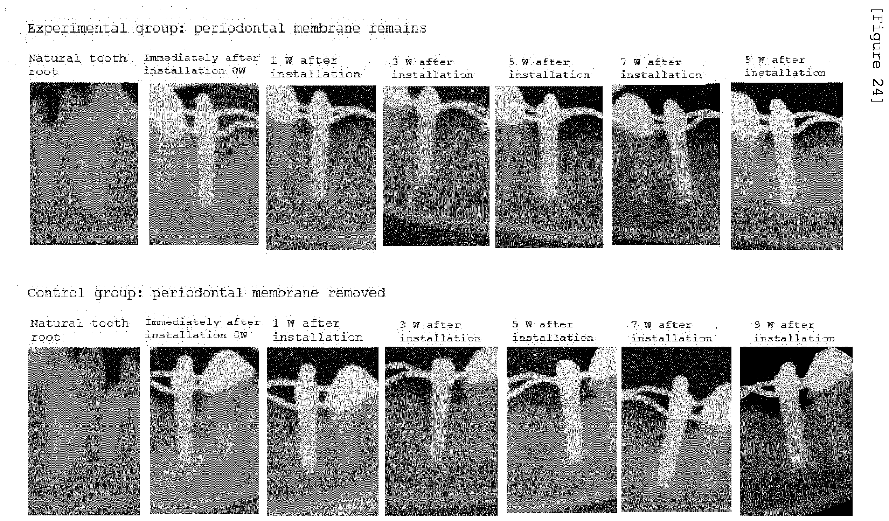

### (5) Result (Figure 24 - Figure 28)

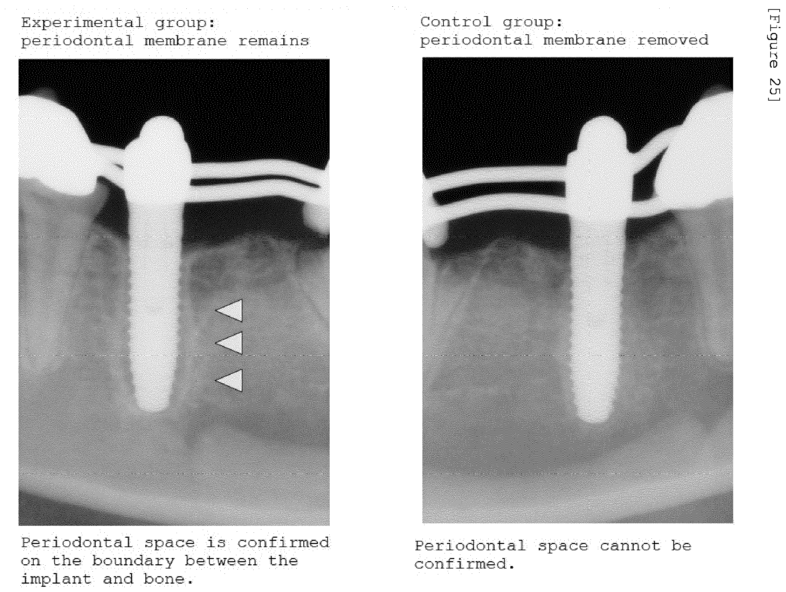

During the observation period of 9 weeks from the start of transplantation to resection, samples showing observation of fall-out or bacterial infection of the implant, or severe inflammation were not seen. From X-ray observation over time, regeneration of the alveolar bone from the inner wall of the transplantation socket side towards the implant side was seen over Weeks 3 - 5 after transplantation. In transplantation Weeks 7-9, periodontal space was seen between the implant surface and the alveolar bone of the inner wall of the transplantation socket, and it was shown to be surviving on the jaw bone. On the other hand, although alveolar bone regeneration around the implant was also seen in the control group having the periodontal membrane tissue completely removed, more distinct periodontal space was seen in the experimental group having healthy periodontal membrane tissue remaining (Figures 24 and 25).

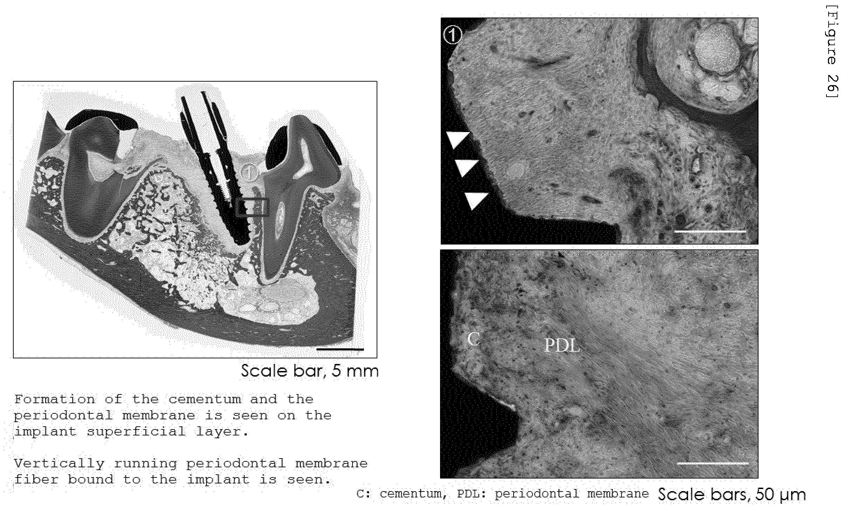

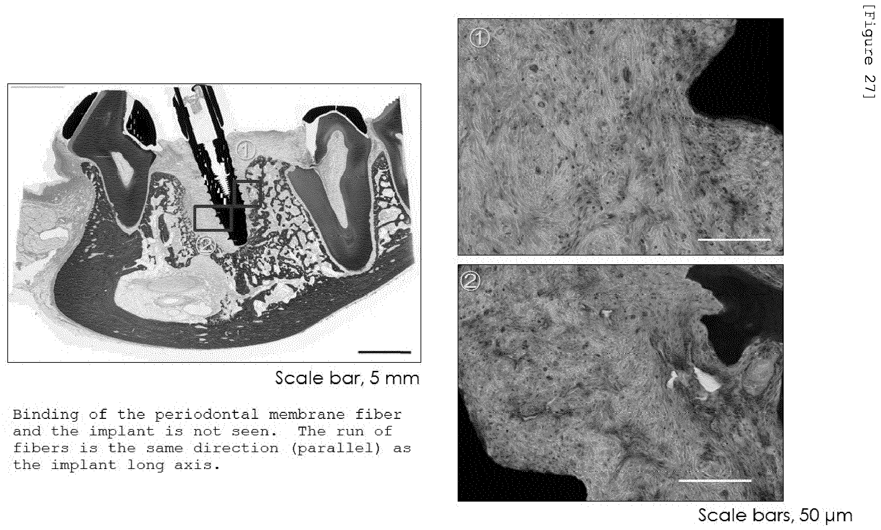

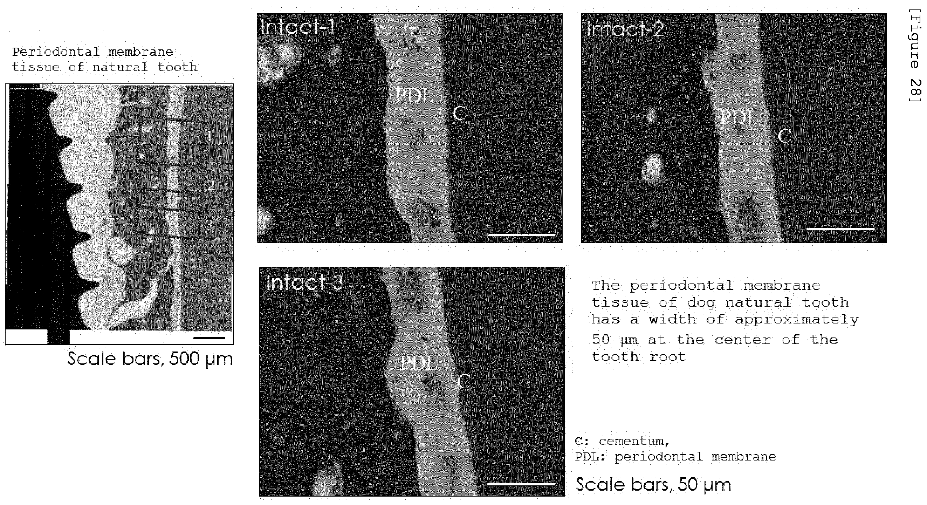

From histological analysis, in the experimental group, periodontium structure equivalent to a natural tooth such as the cementum, the periodontal membrane, and the alveolar bone was seen on the implant superficial layer, showing that it is surviving on the jaw bone. Moreover, the presence of distinctly running periodontal membrane fibers in the vertical direction connecting the implant superficial layer and the surrounding alveolar bone became clear (Figure 26). On the other hand, in the control group, periodontal membrane fibers bound to the implant surface was not confirmed, and a fibrous encapsulated-like tissue structure that runs parallel to the implant long axis was seen (Figure 27) . The tissue image of the periodontal membrane tissue (periodontal membrane that was present from before the experiment) of a natural tooth of a test animal is shown in Figure 28.

From the above results, the practicality of a bioimplant that utilizes healthy periodontal membrane tissue that remained in the extraction socket by rendering physiological occlusion stimulation utilizing a suspension fixed device was also shown in a dog model.

## Claims

1. A dental implant device that allows formation of functional periodontium, **characterized in that**:
said implant device comprises a dental implant body and an implant fixture,
said implant fixture comprises a portion to be fixed to said implant body and a portion to be fixed to a tooth adjacent to the transplantation site of said implant body in a subject, and is configured so that mechanical stimulation from the tooth adjacent to said transplantation site is transmissible to said implant body, and
said implant device is placed into the oral cavity of said subject so that said implant body is inserted at said transplantation site while said implant fixture is fixed to said implant body and the tooth adjacent to said transplantation site.

2. The implant device according to claim 1, **characterized in that** said transplantation site is an extraction socket.

3. The implant device according to claim 2, **characterized in that** said extraction socket is an extraction socket within seven days of tooth extraction.

4. The implant device according to claim 2 or 3, **characterized in that** said implant body is inserted in said extraction socket so as not to contact the bottom of said extraction socket and is fixed by said implant fixture, to thereby maintain a state where said implant body does not contact the bottom of said extraction socket.

5. The implant device according to any one of claims 1 to 4, **characterized in that** said implant fixture is fixed to teeth on both sides adjacent to the transplantation site of said implant body.

6. The implant device according to any one of claims 1 to 5, **characterized in that** the material of said implant fixture is selected from the group consisting of a metal material, a ceramic material, a polymeric material, a hard fiber material, and ebonite.

7. The implant device according to any one of claims 1 to 6, **characterized in that** a periodontal membrane tissue-derived or tooth germ tissue-derived cell mass is placed on all or a part of the surface of said implant body.

8. The implant device according to claim 7, **characterized in that** said tooth germ tissue-derived cell mass is a tooth germ mesenchymal tissue-derived or dental follicle tissue-derived cell mass.

9. The implant device according to claim 7 or 8, **characterized in that** said cell mass is a periodontal membrane tissue-derived cell sheet.

10. The implant device according to any one of claims 1 to 9, **characterized in that** it further comprises a coating layer of a surface coating agent on the whole surface of said implant body or a portion thereof.

11. The implant device according to claim 10, **characterized in that** said surface coating agent is selected from the group consisting of hydroxyapatite, α-tricalcium phosphate, β-tricalcium phosphate, and collagen.

12. A method of transplanting a dental implant to a subject comprising:
(A) a step of preparing a dental implant device that allows formation of functional periodontium,
wherein said implant device comprises a dental implant body and an implant fixture, and
said implant fixture comprises a portion to be fixed to said implant body and a portion to be fixed to a tooth adjacent to the transplantation site of said implant body in a subject, and is configured so that mechanical stimulation from the tooth adjacent to said transplantation site is transmissible to said implant body; and
(B) a step of placing said implant device into the oral cavity of said subject,
wherein said implant device is placed into the oral cavity of said subject so that said implant body is inserted at said transplantation site while said implant fixture is fixed to said implant body and the tooth adjacent to said transplantation site.

13. The transplantation method according to claim 12, **characterized in that** said transplantation site is an extraction socket within seven days of tooth extraction.

14. The transplantation method according to claim 12 or 13, **characterized in that** in said step (B), said implant body is inserted in said extraction socket so as not to contact the bottom of said extraction socket and is fixed by said implant fixture, to thereby maintain a state where said implant body does not contact the bottom of said extraction socket.

15. The transplantation method according to any one of claims 12 to 14, **characterized in that** said subject is a non-human mammal.
